# EUROPEAN PATENT APPLICATION

(11) **EP 3 378 439 A1**
(43) Date of publication of application: **26.09.2018**
(21) Application number: 17162751.6
(22) Date of filing: 24.03.2017
(51) Int. Cl.: A61F 2/14, A61F 9/00

(54) **INTRACORNEAL IMPLANT**

(71) Applicant: KEJAKO SA, 1228 Plan-les-Ouates (CH)
(72) Inventor: ASSOULINE, Michael, 1228 Plan les Ouates (CH); ISARD, Pierre-François, 1228 Plan les Ouates (CH); BOS, Gilles, 1228 Plan les Ouates (CH); ENFRUN, David, 1228 Plan les Ouates (CH); MAURER, Aurélien, 1228 Plan les Ouates (CH)
(74) Representative: KATZAROV S.A.

(57) **Abstract**

The present relates to an intracorneal implant (1) adapted to be inserted within an intrastromal volume (2) of a cornea characterized in that said implant (1) comprises a plurality of particles (11) movable relatively to each other so as to be adapted to be implanted in an intrastromal volume (2) of an eye cornea to provide an annular opaque light barrier, and a biocompatible matrix (12) material wherein said plurality of particles (11) are distributed, characterized in that said particles (11) present a variable opacity adapted to be reversibly modified through activation between at least a transparent state and an opaque state, in that once inserted, a portion of said particles (11) is activated such that the intracorneal implant (1) presents an outer diameter (d₁) of at least equal to a diameter of a dilated pupil and an internal diameter (d₂) sized so as to generate a stenopeic effect, and in that the center of the implant coincides with the center of the pupil.

## Description

### Technical Field

The present invention relates to an intracorneal implant and more particularly to an intracorneal implant for treating presbyopia. Further the present invention relates to intracorneal implant manufacturing and implementing methods.

### Background of the art

The young human eye can naturally focus from far to near objects. The mechanism underlying this focus is called the visual accommodation process. It is composed of three main aspects: first, a convergence of the eye ball as objects get closer, then - for a given luminosity - an increase of pupil constriction, and finally an adjustment of the crystalline lens shape modulated by the ciliary muscle contraction.

However, as the human eye gets older, the accommodation process gets more and more difficult for several reason, this is called presbyopia. Presbyopia is a fairly common condition in people over age 40 that compromises the ability to accommodate for near objects. Presbyopia is experienced as a blurring of the person's vision and frequently arises when gazing near distance objects like reading a book for example or reading or working at a computer. An untreated patient might compensate by moving the viewed material farther away than would be their previous practice. Roots of presbyopia lays in the aging of the crystalline lens that becomes larger and stiffer, and therefore harder to deform while convergence and pupil constriction seems not to be affected.

Use of readers or multifocal eyeglasses is the most common treatment for presbyopia symptoms. Readers have one specific refractive correction for near vision. Multifocal eyeglasses provides both refractive correction for distance viewing and refractive correction for viewing near objects.

In another proposed treatment, corneal inlays, also called corneal implants in the following description, have been implanted in human eyes to attempt to reverse the effects of presbyopia and to restore near and intermediate vision. Corneal inlays are ocular devices that incorporates pinhole imaging and are implanted in the cornea. A pinhole imager has a small aperture through which light is transmitted to the retina. The pinhole imager has the effect of increasing the range of distance from the eye over which objects are in focus, referred to as the depth of focus. Increase in the depth of focus is naturally obtained by pupil constriction, it reduces the eye's need for the normal processes of accommodation, including muscle- induced lens shape change and could therefore be increased with a corneal inlay when the shape of the crystalline lens becomes harder to change.

For example, one available corneal inlay (Kamra inlay from Acufocus) is an opaque circular micro-disc with a small opening in the center. Additionally, there are high precision, laser-etched micro-openings through the depth of the inlay to help maintain a healthy cornea.

The cornea typically includes five major layers with the stroma as the thickest and centrally located layer. The stroma is made of overlying and interlocking collagen fibrils to form layer-like structures. The cornea ideally is transparent and colorless for optimum clarity of vision.

When placed in the cornea, the small opening in the center of the inlay blocks unfocused light and only allows focused light to reach the retina. With focused light rays, patients often enjoy a wider range of improved vision. Other corneal inlays are also utilized without providing pinhole effect but refractive correction. For example, some are small lenses inserted into the cornea to reshape the front surface of the eye to increase optical power of the cornea and improve near vision. Others are multifocal inlays providing, at least, 2 ranges of vision. These corneal inlays are used to improve near vision and to reduce the need for reading glasses in older adults who have presbyopia. For example, one corneal inlay (Vue+ or PresbyLens) is a 2 mm diameter inlay made of hydrogel plastic, which is similar to the material used in soft contact lenses. The inlay improves both near and intermediate vision. The inlay is placed within the cornea under a LASIK-style flap (laser assisted in situ keratomileusis). When in position, the inlay changes the curvature of the cornea so the front of the eye acts like a multifocal contact lens. Patients who had the inlay implanted in the cornea of their non-dominant eye can realize an improvement of five lines of near visual acuity and an improvement of one to two lines of intermediate visual acuity on a standard eye chart, while maintaining binocular distance vision of 20/20. Another example, for the multifocal corneal inlay, is the Icolens from Neoptics: a 3mm diameter inlay with bifocal design comprising a central zone for distance correction and a peripheral positive zone for near vision correction.

Corneal tattooing has also been used separately to conceal corneal scarring or to conceal a white cataract by applying dyes on a cornea that has been cauterized. Other non-perforating micro-needle treatments have been used for the cosmetic treatment of leucoma. Similarly, others have treated the opaque cornea of patients by introducing a dye in a corneal pocket that had been previously pre-dissected. Keratopigmentation has been used to treat iris defects with the use of new pigments and purified and inert dyes that no longer interact with neighboring tissues.

Document US 2015/305927 describes a conventional corneal tattooing for medical purposes which involves the use of a 3D-gauge needle to apply titanium or iron dioxide pigment to the cornea using small punctures to the eye. It typically takes 20 to 30 minutes to make between 200 to 1'000 small needle punctures to place the dye. The procedure is frequently incomplete, with a high re-treatment rate, and it can be painful. The dye coverage applied by the direct injections may also be incomplete.

One of the main problems of the conventional corneal inlays is that they constitute an intrinsic barrier to the nutrients in the cornea. As a matter of fact, the cornea drains a lot of nutrients for the eye and its sclera and inserting a physical implant hinders or even imped a proper nutrient feeding to the various part of the eye.

There is therefore a need for a corneal inlay adapted for treating presbyopia which would be able to prevent any nutrient barrier effect once installed in the eye.

In this regard, a primary object of the invention is to solve the above-mentioned problems and more particularly to provide an intracorneal implant adapted for treating presbyopia which would be able to prevent any nutrient barrier effect once installed in the eye.

However, this kind of inlay shall be difficult to implant within the eye and more particularly, in order to provide a perfect stenopeic effect, it is mandatory to have an implantation process providing a perfect centering of the inlay in the eye as well as a perfect size of the inlay with respect to the eye.

Another object of the invention it to provide an intracorneal implant adapted for treating presbyopia which is easy to manufacture and to install in the eye.

A further object of the invention it to provide an intracorneal implant implantation method easy and rapid to carry out which is adjustable to each specific patient's eye in size.

### Summary of the invention

The above problems are solved by the present invention.

A first aspect of the invention is an intracorneal implant adapted to be inserted within an intrastromal volume of a cornea characterized in that said implant comprises a plurality of particles movable relatively to each other so as to be adapted to be implanted in an intrastromal volume of an eye cornea to provide an annular opaque light barrier, and a biocompatible matrix material wherein said plurality of particles are distributed, characterized in that said particles present a variable opacity adapted to be reversibly modified through activation between at least a transparent state and an opaque state, in that once inserted, a portion of said particles is activated such that the intracorneal implant presents an outer diameter of at least equal to a diameter of a dilated pupil and an internal diameter sized so as to generate a stenopeic effect, and in that the center of the implant coincides with the center of the pupil. Thanks to this implant, an inlay providing a stenopeic effect to correct presbyopia symptoms while avoiding the usual nutriments obstructions due to its presence is provided thanks to the possible movement of the plurality of particles. Further, the activable particles permits to generate an inlay perfectly centered on the pupil and perfectly sized to an eye of each patient.

According to a preferred embodiment of the present invention, the plurality of particles are stained with a biocompatible reversible dye. In this manner, the opacity of the plurality of particles is easy to obtain and to vary without the need of a further surgical step.

Advantageously, the plurality of particles have a size in the range from 15 microns to 30 microns. This specific range is particularly adapted for an intrastromal inlay as it permits to inject more particles thereby providing a better elasticity between the particles. Also, smaller particles means smaller cannula and therefore smaller incision for injection.

Preferably, the plurality of particles are made of a biocompatible material. Thus, there is a lower risk for inflammatory reaction and no need to replace them too often.

According to a preferred embodiment of the present invention, the biocompatible material is selected from the group of monomers comprising at least one of a hydrophilic acrylic monomer for example EMA, MMA or HEMA, or a vinylpyrrolidone for example povidone, PV, PVP or a hydrophobic monomer for example PMMA or EGDMA, and one cross-linker. These materials have shown particularly good results since hydrophilic materials easily absorb the dye while a hydrophobic material shall be pigmented within its bulk.

Advantageously, the plurality of particles are subjected to a surface treatment with heparin or Hyaluronic Acid. This provides a further excellent anti-agglomeration and repelling properties to the plurality of particles improving the movable property of the plurality of particles.

Preferably, the number of particles comprised in the implant is in the range from 50'000 particles to 1'500'000 particles. This particular range, which depends from sizes of the particles and final size of the implant, has produced excellent intrastromal inlays as the more particles one inject the better elasticity between the particles we provide.

According to a preferred embodiment of the present invention, the matrix material is at least one of a viscoelastic gel, viscous ophthalmic serum or any suitable carrier. Such a matrix will provide the inlay with a proper cohesive inlay while permitting the relative movement of the plurality of particles.

Advantageously, the plurality of particles are microspheres. This form is particularly adapted for a movement between the particles due to the absence of edges or corners.

Preferably, upon implantation and activation in an intrastromal volume of an eye cornea, said plurality of particles is adapted to form a single or multiple layered barrier having an annular shape with an internal diameter of 1.6 mm and an outer diameter of, at least, 4 mm. Therefore, once in place, the inlay will provide an intracorneal implant with a pinhole so as to artificially reduce the pupil diameter and therefore increase the depth of focus to reduce presbyopia symptoms.

A second aspect of the invention is an intracorneal implant delivery device comprising a vessel in which the intracorneal implant according to the first aspect of the invention is housed and a delivery tube wherein the internal diameter of the lumen is higher than the diameter of the particles of the implant and characterized in that said device is mounted on a sterile ready-to use syringe adapted for injecting intracorneal implant within an intrastromal volume of an eye cornea to provide an annular light barrier having an outer diameter of at least equal to the diameter of the dilated pupil and an internal diameter sized so as to generate a stenopeic effect. The particular advantages of this device of the invention being similar to the ones of the intracorneal implant of the first aspect of the invention, they will not be repeated here.

A third aspect of the invention is an intracorneal implant implanting method for implanting an intracorneal implant according the first aspect of the invention, characterized in that said method comprises the steps of creating an intrastromal volume within the eye cornea, inserting said intracorneal implant within said intrastromal volume, closing said intrastromal volume, and activating at least a portion of said particles so as to generate an intracorneal implant presenting an outer diameter of at least equal to a diameter of a dilated pupil and an internal diameter sized so as to generate a stenopeic effect, and in that the center of the implant coincides with the center of the pupil. The particular advantages of this device of the invention being similar to the ones of the intracorneal implant of the first aspect of the invention, they will not be repeated here.

According to a preferred embodiment of the present invention, the intrastromal volume is created through the use of a laser and could be advantageously coupled to a SMILE® or comparable procedure. Given the background of this techniques, the process will therefore be safe.

Advantageously, the intrastromal volume has an annular shape having an internal diameter of 1.6 mm and an outer diameter of, at least, 4 mm. Therefore, the volume will provide a proper space for the intracorneal implant with a pinhole so as to artificially reduce the pupil diameter and therefore increase the depth of focus to limit the presbyopia.

Preferably, the volume of the intrastromal volume is ranging from 0.5 to 5 µl. Such a volume will permits to install a proper inlay.

### Brief description of the drawings

Further particular advantages and features of the invention will become more apparent from the following non-limitative description of at least one embodiment of the implant, the device and the implantation process of the invention which will refer to the accompanying drawings, wherein
- Figure 1 represents a schematic view of a first aspect of the present invention;
- Figure 2 represents a schematic view of a second aspect of the present invention;
- Figures 3A to 3C schematically represent the three steps of the implantation process; and
- Figures 4A to 4D schematically represent the different steps of the activation process.

### Detailed description of the invention

The present detailed description is intended to illustrate the invention in a non-limitative manner since any feature of an embodiment may be combined with any other feature of a different embodiment in an advantageous manner.

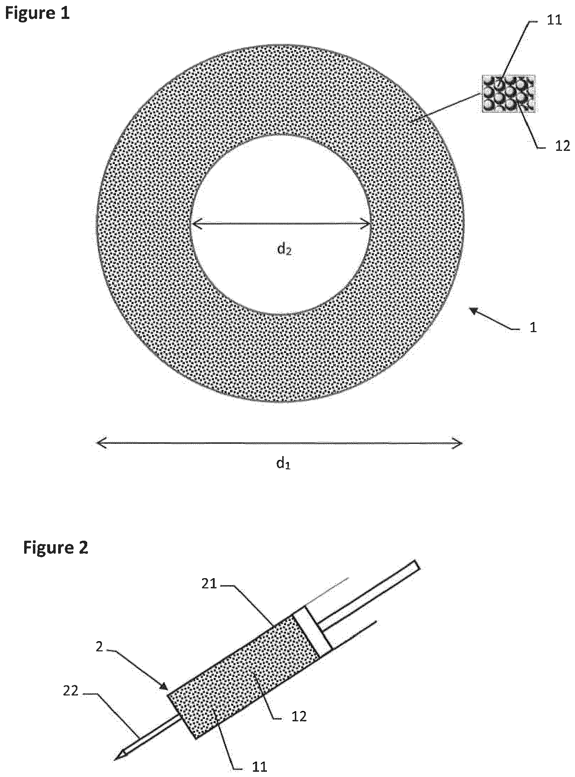

Figure 1 shows the first aspect of the invention which is an intracorneal implant 1, also called inlay, adapted to be inserted within an intrastromal volume 3 of a cornea. The said volume may have the form of a channel or preferably of a tunnel. As one can see, in this embodiment, the implant 1 comprises a plurality of particles 11, preferably in the range from 50'000 particles to 1'500'000 particles have a size in the range from 15 microns to 30 microns, made of a biocompatible material which are preferably stained with a biocompatible dye and are movable relatively to each other. Advantageously, the plurality of particles 11 are microspheres, this form is particularly adapted for a movement between the particles 11 due to the absence of edges or corners. The term microsphere shall however not be strictly considered here since exact spherical shape is hard to obtained and elliptical shape shall be considered within the "microsphere" term, for example.

Also, according to the first aspect of the invention, the particles (11) present a variable opacity adapted to be reversibly modified through activation between at least a first opacity state and a second opacity state. One shall understand here that the first opacity may be a fully transparent state or a fully opaque state, the second state being the inverse. Once inserted, a portion of said particles (11) is activated such that the intracorneal implant (1) presents an outer diameter (d₁) of at least equal to a diameter of a dilated pupil and an internal diameter (d₂) sized so as to generate a stenopeic effect, and in that the center of the implant coincides with the center of the pupil.

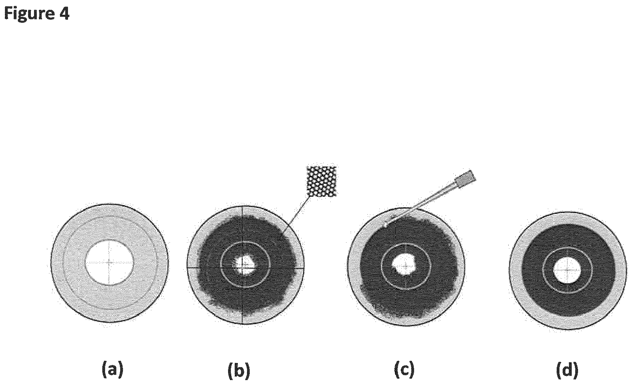

Activation can therefore mean transforming the particles from opaque to transparent like shown in the figures 4A-4D. In this case one should activate the particles outside the outer diameter (d₁) of at least equal to a diameter of a dilated pupil and inside the internal diameter (d₂) sized so as to generate a stenopeic effect. On the other hand, activation can mean transforming the particles from transparent to opaque. This case, not shown in the figures, would mean one should activate the particles inside the outer diameter (d₁) of at least equal to a diameter of a dilated pupil and outside the internal diameter (d₂) sized so as to generate a stenopeic effect.

Activation can be done through different process. For example thanks to the materials or meta materials of the microspheres which optical properties (e.g. transparency) could change depending on their macroscopic or microscopic structure; the latter being adjusted through external energetic beam which will provide permanent or non-permanent changes in the organization (geometry, orientation) of the particles or the bulks of particles creating the appropriate optical filter

The biocompatible material mentioned above is preferably selected from the group of monomers comprising at least one of a hydrophilic acrylic monomer for example EMA, MMA or HEMA, or a vinylpyrrolidone for example povidone, PV, PVP or a hydrophobic monomer for example PMMA or EGDMA, and one cross-linker. These materials have shown particularly good results since hydrophilic materials easily absorb the dye. On the other hand it is possible to use a hydrophobic material like PMMA, or any suitable material, but with this type of material the particle shall be pigmented within its bulk directly.

According to further preferred embodiment of the invention, the plurality of particles 11 are subjected to a surface treatment, preferably with heparin or hyaluronic acid or any suitable material. This provides a further excellent anti-agglomeration and repelling properties to the particles 11 improving the movable property of the particles.

In order to provide a cohesive implant, the plurality of particles 11 are supported by a proper carrier 12. This means that the implant 1 further comprises a biocompatible matrix material 12 which is at least one of a viscoelastic gel, a viscous ophthalmic serum or any suitable carrier, wherein the plurality of particles 11 are distributed in order to stay together to provide an annular opaque light barrier while permitting a relative movement between the plurality of particles 11 to avoid any nutriments obstructions. Such a matrix 12 will provide the inlay 1 with a proper cohesive inlay while permitting the relative movement of the plurality of particles.

The above intracorneal implant 1 is to be implanted in an intrastromal volume 3 of an eye cornea to provide having an outer diameter of at least equal to the diameter d₁ of the dilated pupil, preferably, at least, 4 mm and an internal diameter d₂ sized so as to generate a stenopeic effect preferably 1.6 mm. Thanks to this implant 1, an opaque inlay providing a stenopeic effect to prevent presbyopia while due to its presence is provided thanks to the possible movement of the particles.

In Figure 2 is represented a second aspect of the invention which is an intracorneal implant delivery device 2 comprising a vessel 21 in which the intracorneal implant 1 described above is housed in an injectable form and a delivery tube 22 wherein the internal diameter of the lumen is higher than the size/diameter of the particles 11 of the implant 1. This device 2 is adapted to be mounted on a sterile ready-to use syringe adapted for the injecting intracorneal implant 1 within an intrastromal volume 3 of an eye cornea to provide an annular opaque light barrier having an outer diameter of at least equal to the diameter of the pupil and an internal diameter sized so as to generate a stenopeic effect.

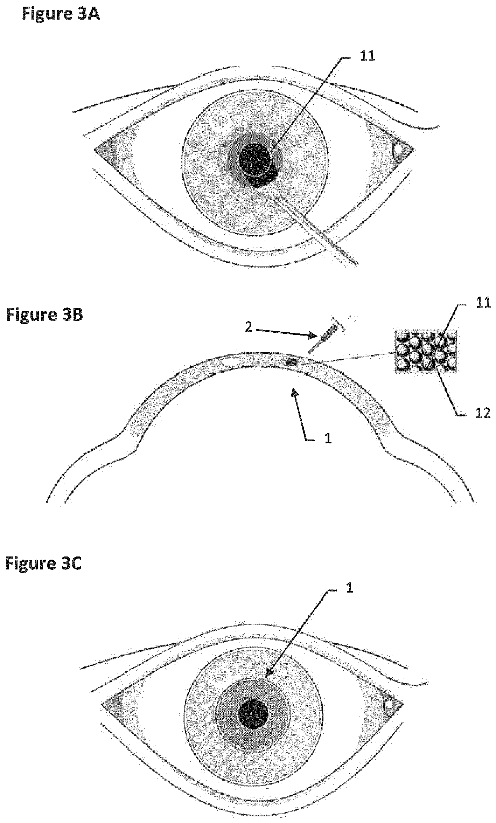

Figures 3A to 3C show the third aspect of the invention which is an intracorneal implant implanting method for implanting an intracorneal implant 1 of the first aspect of the invention.

The method comprises a first steps consisting in creating an intrastromal volume 3 within the eye cornea. Preferably, the intrastromal volume 3 has an annular shape and is provided concentrically around the pupil axis.

There are several ways to carry out this process, a preferred technique, actually schematically shown in figure 3A which actually shows the lenticule extraction, consists in cutting a annular lenticule with a laser such as a femtosecond laser or an excimer laser, then the lenticule is extracted through a small incision thereby creating an intrastromal volume 3. The process can actually be done directly through photo-ablation of the target zone or as shown through excision of the lenticule which is thereafter extracted from the target zone. As a preferable option, one can use a SMILE® process (Small Incision Lenticule Extraction) using a femtosecond laser.

Preferably, the intrastromal volume 3 has an annular shape having an internal diameter of 1.6 mm and an outer diameter of 4 mm. This particular inner diameter has shown a stenopeic effect/pinhole effect while the outer diameter approximately corresponds to a diameter slightly larger than the largest pupil diameter such that the barrier is large enough to prevent light from passing even when the pupil enlarges its diameter. The second step, actually schematically shown in figure 3B, consists in inserting the intracorneal implant within the intrastromal volume thanks to the device 2, preferably through the small incision used to extract the lenticule, which preferably presents a volume ranging from 0.5 to 5 µl. Such a volume will permits to install a proper inlay. A third a last step consists in closing the intrastromal volume 3. Figure 3C shows the eye with the intracorneal implant in position and when the third step of closing the intrastromal volume 3 is terminated.

The last step, which could however be carried out before the intrastromal volume closing step if needed, consists in activating at least a portion of said particles so as to generate an intracorneal implant (1) presenting an outer diameter (d₁) of at least equal to a diameter of a dilated pupil and an internal diameter (d₂) sized so as to generate a stenopeic effect, and in that the center of the implant coincides with the center of the pupil.

### Examples

Several corneal inlays have been generated with different sizes and number of particles. Table 1 below shows four examples (examples 1-4) which all showed improved stenopeic property with a high nutrient passing property. In the table, all sizes are in millimeters.

**Table 1- Examples 1-5**

| | Example1 | Example2 | Example3 | Example4 | Example5 |
|---|---|---|---|---|---|
| Ext. Diam. Inlay (mm) | 4.00 | 4.00 | 4.00 | 4.00 | 4.00 |
| Int. Diam. Inlay (mm) | 1.60 | 1.60 | 1.60 | 1.60 | 1.60 |
| Layers (numbers) | 2.00 | 2.00 | 3.00 | 5.00 | 2.00 |
| Radius beads (mm) | 0.005 | 0.010 | 0.005 | 0.005 | 0.015 |
| Beads volume (µl) | 0.000001 | 0.000004 | 0.000001 | 0.000001 | 0.000014 |
| Thickness inlay (mm) | 0.019 | 0.037 | 0.027 | 0.045 | 0.056 |
| Volume to fill (µl) | 0.79 | 1.58 | 1.15 | 1.88 | 2.36 |
| beads/fluid ratio | 0.40 | 0.40 | 0.40 | 0.40 | 0.40 |
| Number of beads | 601'905 | 150'476 | 881'250 | 1'439'941 | 66'878 |

While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the spirit and scope of this disclosure. This for example particularly the case regarding the different apparatuses which can be used with the intracorneal implant delivery device 2 or the different cohesive gel which could be used as a matrix, etc.

## Claims

1. Intracorneal implant (1) adapted to be inserted within an intrastromal volume (2) of a cornea **characterized in that** said implant (1) comprises
a plurality of particles (11) movable relatively to each other so as to be adapted to be implanted in an intrastromal volume (2) of an eye cornea to provide an annular opaque light barrier, and
a biocompatible matrix (12) material wherein said plurality of particles (11) are distributed, **characterized in that**
said particles (11) present a variable opacity adapted to be reversibly modified through activation between at least a transparent state and an opaque state,
**in that** once inserted, a portion of said particles (11) is activated such that the intracorneal implant (1) presents an outer diameter (d₁) of at least equal to a diameter of a dilated pupil and an internal diameter (d₂) sized so as to generate a stenopeic effect, and **in that** the center of the implant coincides with the center of the pupil.

2. Intracorneal implant according to claim 1, **characterized in that** said plurality of particles (11) are stained with a biocompatible reversible dye.

3. Intracorneal implant according to claim 1 or 2, **characterized in that** said plurality of particles (11) have a size in a range from 15 microns to 30 microns.

4. Intracorneal implant according to any one of claims 1 to 3, **characterized in that** said plurality of particles (11) are made of a biocompatible material.

5. Intracorneal implant according to any one of claims 4, **characterized in that** said the biocompatible material is selected from the group of monomers comprising at least one of a hydrophilic acrylic monomer for example EMA, MMA or HEMA, or a vinylpyrrolidone for example povidone, PV, PVP or a hydrophobic monomer for example PMMA or EGDMA, and one cross-linker.

6. Intracorneal implant according to any one of claims 1 to 5, **characterized in that** said plurality of particles (11) are subjected to a surface treatment with heparin or Hyaluronic Acid.

7. Intracorneal implant according to any one of claims 1 to 6, **characterized in that** the number of particles (11) comprised in said implant (1) is in a range from 50'000 particles to 1'500'000 particles.

8. Intracorneal implant according to any one of claims 1 to 7, **characterized in that** said biocompatible matrix material (12) is at least one of a viscoelastic gel, viscous ophthalmic serum or any suitable carrier.

9. Intracorneal implant according to any one of claims 1 to 8, **characterized in that** said plurality of particles (11) are microspheres.

10. Intracorneal implant according to any one of claims 1 to 9, **characterized in that** upon implantation and activation in the intrastromal volume (3) of an eye cornea, said plurality of particles (11) is adapted to form a single or multiple layered opaque barrier having an annular shape with an internal diameter (d₂) of 1.6 mm and an outer diameter (d₁) of 4 mm.

11. Intracorneal implant delivery device (2) comprising a vessel (21) in which the intracorneal implant (1) of any one of claims 1-10 is housed and a delivery tube (22) wherein an internal diameter of a lumen of said delivery tube (22) is higher than the size of said particles (11) of said implant (1) and **characterized in that** said device (2) is mounted on a sterile ready-to use syringe adapted for injecting said intracorneal implant (1) within an intrastromal volume (3) of an eye cornea to provide an annular opaque light barrier having an outer diameter of at least equal to the diameter of the pupil and an internal diameter sized so as to generate a stenopeic effect

12. Intracorneal implant implanting method for implanting an intracorneal implant according to any one of claims 1 to 10, **characterized in that** said method comprises the steps of
creating an intrastromal volume (3) within the eye cornea,
inserting said intracorneal implant (1) within said intrastromal volume,
closing said intrastromal volume (3), and
activating at least a portion of said particles so as to generate an intracorneal implant (1) presenting an outer diameter (d₁) of at least equal to a diameter of a dilated pupil and an internal diameter (d₂) sized so as to generate a stenopeic effect, and **in that** the center of the implant coincides with the center of the pupil.

13. Intracorneal implant implanting method of claim 12, **characterized in that** said intrastromal volume (3) is created through the use of a laser.

14. Intracorneal implant implanting method of claim 12 or 13, **characterized in that** said intrastromal volume (3) has an annular shape having an internal diameter of 1.6 mm and an outer diameter of, at least, 4 mm.

15. Intracorneal implant implanting method of any of claims 12 to 14, **characterized in that** the volume of said intrastromal volume (3) is ranging from 0.5 to 5 µl.
